# EUROPÄISCHE PATENTSCHRIFT

(11) **EP 1 240 342 B1**
(45) Veröffentlichungstag und Bekanntmachung des Hinweises auf die Patenterteilung: **11.10.2006**
(21) Anmeldenummer: 00991182.7
(22) Anmeldetag: 13.12.2000
(51) Int. Cl.: C12N 15/82

(54) **HERSTELLUNG TRANSGENER PFLANZEN DER GATTUNG TAGETES**
PRODUCTION OF TRANSGENIC PLANTS OF THE TAGETES SPECIES
PRODUCTION DE PLANTES TRANSGENIQUES DE L'ESPECE DES TAGETES

(30) Priorität: 21.12.1999 DE 19962133
(43) Veröffentlichungstag der Anmeldung: 18.09.2002
(73) Patentinhaber: Sungene GmbH & Co. KGaA, 06466 Gatersleben (DE)
(72) Erfinder: KUNZE, Irene, 06466 Gatersleben (DE); HERBERS, Karin, 06484 Quedlinburg (DE); HEIM, Ute, 06466 Gatersleben (DE)
(74) Vertreter: Meyer, Udo
(86) Internationale Anmeldenummer: PCT/EP2000/012643
(87) Internationale Veröffentlichungsnummer: WO 2001/046445

(56) Entgegenhaltungen:
- WALDEN R ET AL: "Gene-transfer and plant-regeneration techniques" TRENDS IN BIOTECHNOLOGY, ELSEVIER, AMSTERDAM, NL, Bd. 13, Nr. 9, September 1995 (1995-09), Seiten 324-331, XP004207196 ISSN: 0167-7799
- ZUKER A ET AL: "Genetic engineering for cut-flower improvement" BIOTECHNOLOGY ADVANCES, ELSEVIER PUBLISHING, BARKING, GB, Bd. 16, Nr. 1, 1998, Seiten 33-79, XP004099136 ISSN: 0734-9750
- DATABASE BIOSIS [Online] BIOSCIENCES INFORMATION SERVICE, PHILADELPHIA, PA, US; 10. April 1997 (1997-04-10) NAGARAJU V ET AL: "Agrobacterium-mediated genetic transformation in Gerbera hybrida." Database accession no. PREV199800433783 XP002180979 & CURRENT SCIENCE (BANGALORE), Bd. 74, Nr. 7, 10. April 1997 (1997-04-10), Seiten 630-634, ISSN: 0011-3891

## Beschreibung

Die Erfindung betrifft ein Verfahren zur Herstellung stabil transformierter, fertiler Pflanzen der Gattung Tagetes, dadurch gekennzeichnet, daß folgende Schritte enthalten sind: (a) Anzucht der zu transformierenden Ausgangspflanze sowie Gewinnung des geeigneten Explantates, (b) Transfer von DNA-Sequenzen in Pflanzenzellen, (c) Selektion transformierter Pflanzenzellen und (d) Regeneration transgener, fertiler Pflanzen, wobei für die Regeneration in der Phase 1 ein Cytokinin bzw. cytokininkonjugat und ein Auxin bzw. Auxinkonjugat und in der phase 2 ein Auxin bzw. Auxinkonjugat und Gibberelinsäure GA3 eingesetzt werden. Darüberhinaus ist das Verfahren dadurch gekennzeichnet, daß der Transfer von DNA-Sequenzen in die zu transformierende Pflanze bzw. deren Explantate beispielsweise durch Agrobacterium tumefaciens vermittelt werden kann. Desweiteren ist das Verfahren dadurch gekennzeichnet, daß Blätter als Explantate genutzt werden können, daß eine 2 bis 8 tägige Co-Kultivierung mit Agrobacterium erfolgt und daß während der Co-Kultivierung ein Medium verwendet wird, das Wachstumsregulatoren enthält. Das Verfahren ist weiterhin dadurch gekennzeichnet, daß für die Selektion beispielsweise Phosphinothricin eingesetzt werden kann und daß für die Regeneration zu intakten transgenen Pflanzen zwei verschiedene Kombinationen an Wachstumsregulatoren verwendet werden. Das Verfahren ist dazu geeignet, die Art Tagetes erecta bzw. Tagetes patula zu transformieren. Die Erfindung betrifft auch transgene, fertile Tagetespflanzen selbst, die durch das beschriebene Verfahren hergestellt worden sind sowie deren transgene Samen.

Die zur Familie der Asteraceae gehörende Pflanzengattung Tagetes besitzt etliche interessante Eigenschaften, was ihre Nutzung sowohl als Kulturpflanze als auch als Zierpflanze erklärt. Viele Arten dieser Gattung produzieren bioaktive Verbindungen mit nematozider, fungizider und insektizider Wirkung, akkumulieren Flavonoide und Karotinoide mit antioxidativen und farbgebenden Aktivitäten, sind Salz-resistent und dienen auf Grund ihrer vielfältigen Blütenfarben und -formen dekorativen Zwecken.

Die nematozide Wirkung der Tageteswurzeln beruht auf der Synthese von Thiophenderivaten. Thiophenderivate sind heterozyklische, schwefelhaltige Verbindungen, die vor allem in Wurzeln und Hypokotylen akkumulieren. Sie sind durch einen 5-Ring gekennzeichnet, der ein S-Atom enthält. An der Ringbildung ist eine aus der Aminosäure Cystein stammende Sulfhydrylgruppe beteiligt. Wichtige Vertreter der Thiophene, die auch in Tagetes gebildet werden, sind z.B.: BBT (5-(but-3-en-1-ynyl)-2,2'-bi-thienyl), BBTOAc (5-(4-acetoxy-1-butynyl)-2,2'-bithienyl), BBTOH (5-(4-hydroxy-1-butinyl)-2,2'-bithienyl) und α-T (2,2':5',2"-terthienyl).

Die Blütenblätter von Tagetes enthalten 9 - 22 % Flavonoide und ca. 27 % Carotinoide, die zu 0,4 % aus β-Caroten, zu 1,5 % aus Cryptoxanthinester und zu 86,1 % aus Xanthophyllestern bestehen (Benk et al., Riechst, Aromen und Koerpen, 26 (1976), 216-221).

Nach einer Schätzung aus dem Jahre 1975 gibt es mehr als 2000 verschiedene Flavonoide. Einige wichtige Vertreter sind z.B. die Anthocyanine mit 250, die Chalcone mit 60, die Aurone mit 20 und die Flavone mit 350 bekannten Strukturen, die alle farbgebende Eigenschaften besitzen. Flavonole mit 350 und den Isoflavonoiden mit 15 Vertretern verleihen den Pflanzen Eigenschaften wie Fraßschutz oder haben eine toxische Wirkung auf Pilze.

Carotinoide gehören zu der großen Gruppe der Terpenoide. Die meisten von ihnen sind Tetraterpene. Carotinoid-Kohlenwasserstoffe heißen auch Carotene und ihre oxydierten Derivate sind die Xanthophylle. Carotinoide sind essentielle Komponenten in photosynthetisch aktiven Membranen aller Pflanzen, Algen und Cyanobakterien. Sie sind in den Pigmentsystemen (Lichtfallen) der Chloroplasten enthalten und wirken am Prozeß der primären Lichtabsorption und Photonenkanalisation der Photosynthese mit. Darüber hinaus nehmen sie die Funktion von Lichtrezeptoren bei einer Reihe weiterer lichtinduzierter Prozesse in der Pflanze wahr. Die gelbe Farbe vieler Blüten geht auf carotinoidhaltige, meist chlorophyllfreie Chromoplasten zurück. Pflanzliche Carotinoide dienen auch als Vorstufen für die Biosynthese des pflanzlichen Wachstumsregulators Abscisinsäure sowie des Vitamin A, das für die Ernährung des Menschen und der Tiere bedeutsam ist. Es erlangt als potentielles Antikrebsmittel wachsendes Interesse und wird als Farbstoff in der Kosmetik und Nahrungsmittelindustrie eingesetzt. Carotinoide aus getrockneten und gemörserten Blütenblättern von Tagetes finden als Geflügelfutterzusatz bereits für die farbliche Intensivierung des Hühnereigelbs Verwendung.

Gegenwärtig besteht großes wirtschaftliches Interesse, den Carotinoidgehalt und die Carotinoidzusammensetzung in Pflanzen zu erhöhen bzw. zu verbessern.

Die begrenzte genetische Vielfalt in bekannten Tagetes Sorten schränkt die Möglichkeiten der Verbesserung dieser Sorten mit Hilfe klassisch biologischer, züchterischer Methoden stark ein.

Mittels gentechnischer Verfahren ist es möglich, gezielt Fremdgene in das Pflanzengenom zu übertragen. Dieser Prozeß wird als Transformation und die resultierenden Pflanzen als transgen bezeichnet. Grundvoraussetzung für die Erzeugung transgener Pflanzen ist die Verfügbarkeit eines geeigneten Transformationssystems, das Vorhandensein eines selektionierbaren Markers, die Identifizierung erfolgreich transformierter Pflanzenzellen und die Regeneration der transformierten Zellen zu ganzen fertilen Pflanzen.

Zur Transformation stehen derzeit mehrere Verfahren zur Verfügung. Die am häufigsten eingesetzte Methode zur Transformation dikotyledoner Pflanzen ist der durch Agrobakterium tumefaciens vermittelte Gentransfer. Hierbei wird die natürliche Fähigkeit des Bodenbakteriums ausgenutzt, genetisches Material in das pflanzliche Genom zu integrieren. Weitere geeignete Verfahren sind beispielsweise die Protoplasten-Transformation durch Polyethylenglykol-induzierte DNA-Aufnahme, die Elektroporation, die Sonikation oder Mikroinjektion sowie die Transformation intakter Zellen oder Gewebe durch Mikro- oder Makroinjektion in Gewebe oder Embryonen, Gewebeelektroporation, Inkubation trockener Embryonen in DNA-haltiger Lösung, die Vakuuminfiltration von Samen und der biolistische Gentransfer.

Die Anwendung von Agrobacterium tumefaciens für die Transformation von Pflanzen unter Verwendung von Gewebekulturexplantaten wurde von Horsch et al. (Science 228(1985), 1229-1231), Fraley et al. (Proc. Natl. Acad. Sci. USA 80(1983), 4803-4807) und Bevans et al. (Nature 304(1983), 184-187) beschrieben. Viele Stämme von Agrobacterium tumefaciens sind in der Lage, genetisches Material auch auf Tagetesspecies zu übertragen, wie z.B. die Stämme EHA101 [pEHA101], EHA105[pEHA105], LBA4404[pAL4404], C58C1[pMP90] und C58C1[pGV2260]. Der Stamm EHA101[pEHA101] wurde von Hood et al. (J. Bacteriol. 168(1986), 1291-1301), der Stamm EHA105[pEHA105] von Hood et al. (Transgenic Research 2 (1993), 208-218), der Stamm LBA4404[pAL4404] von Hoekema et al. (Nature 303(1983), 179-181), der Stamm C58C1[pMP90] von Koncz and Schell (Mol. Gen. Genet. 204(1986), 383-396) und der Stamm C58C1[pGV2260] von Deblaere et al. (Nucl. Acids Res. 13(1985), 4777-4788) beschrieben.

Der für die Transformation eingesetzte Agrobakterienstamm enthält zusätzlich zu seinem entwaffneten Ti-Plasmid ein binäres Plasmid mit der zu übertragenden T-DNA, die in der Regel ein Gen für die Selektion der transformierten Zellen und das zu übertragende Gen enthält. Beide Gene müssen mit transkriptionalen und translationalen Initiations- und Terminationssignalen ausgestattet sein.

Das Binärplasmid kann beispielsweise durch Elektroporation oder andere Transformationsmethoden in den Agrobakterienstamm übertragen werden (Mozo & Hooykaas, Plant Mol. Biol. 16 (1991), 917-918). Die Cokultur der pflanzlichen Explantate mit dem Agrobakterienstamm findet in der Regel für zwei bis drei Tage statt.

Die Expression von Fremdgenen kann konstitutiv, induzierbar (durch biotische und abiotische Faktoren), gewebespezifisch bzw. entwicklungsspezifisch reguliert sein. Eine relativ konstitutive Expression wird beispielsweise durch den 35S Promotor des Cauliflower Mosaik Virus in Pflanzen erreicht, die von Shewmaker et al. (Virology 140(1985), 281-288) und Gardner et al. (Plant Mol. Biol. 6(1986), 221-228) beschrieben worden ist.

Als selektierbare Markergene können beispielsweise das Bialophosresistenzgen (bar), das Kanamycin- bzw. G418- Resistenzgen (NPTII) sowie das DOG^{R}1-Gen verwendet werden. Das Bialophosresistenzgen wurde ursprünglich von Streptomyces hygroscopicus isoliert. Es codiert für eine Phosphinothricinacetyltransferase (PAT), die die freie Aminogruppe von Phosphinothricin (PPT) acetyliert und damit eine Detoxifizierung des PPT erreicht (de Block et al., EMBO J. 6 (1987), 2513-2518). Das NPTII Gen codiert für eine Neomycinphosphotransferase, die durch eine Phosphorylierungsreaktion die inhibierende Wirkung von Kanamycin, Neomycin, G418 und Paromomycin reduziert. Das Gen DOG^{R}1 wurde aus der Hefe Saccharomyces cerevisiae isoliert (Sonnewald und Ebneth, EP 0 807 836). Es codiert für eine 2-Desoxyglukose-6-phosphat Phosphatase, die Resistenz gegenüber 2-DOG verleiht (Randez-Gil et al., Yeast 11(1995), 1233-1240).

Bisher wurde die Gattung Tagetes trotz ihrer interessanten biotechnologischen Eigenschaften hinsichtlich gentechnischer Modifikationen kaum bearbeitet. Voraussetzung für eine gentechnische Verbesserung von Tagetes ist ein gutes Regenerationssystem. Regeneration durch Organogenese unter Verwendung von Blättern als Ausgangsexplantat wurde von Ketel (Physiol. Plant. 93 (1986), 298-304), Khotari und Chandra (Hortscience 19(1984a), 703-705) und (J. Plant Physiol. 122(1986), 235-241) entwickelt. Khotari und Chandra beschrieben auch die Regeneration von Pflanzen aus unreifen, unbefruchteten Blütenscheiben (J. Plant Physiol. 117(1984b), 105-108). Auch Hypokotyle können für die Regeneration verwendet werden (Belarmino et al., Jpn J. Breed 42 (1992), 835-841).

Die Transformation und Etablierung von Wurzelkulturen von Tagetes laxa ist von Talou et al., Planta Médica 60, 260-262(1994) beschrieben worden, doch Transformationsverfahren mit einer sich anschließenden Regeneration zu transgenen, fertilen Pflanzen sind bisher für die Gattung Tagetes nicht beschrieben.

Andere bekannte Gattungen der Familie Asteraceae sind beispielsweise Taraxacum mit der bekannten Art Taraxacum officinale (Löwenzahn), Dahlia, Helianthus mit der bekannten Art Helianthus annuus (Sonnenblume), Aster, Calendula (Ringelblume), Carduus (einfacher Pappus), Cichorium u.a.. Mit Ausnahme der Sonnenblume existieren für sämtliche Gattungen dieser Familie keine oder nur unzureichende in vitro Techniken oder sonstige Techniken, die zur Transformation dieser Spezies geeignet wären. Obwohl die einzelnen Vertreter der Familie der Asteraceae morphologisch relativ einheitlich sind, sind in vitro Techniken zur Regeneration und Transformation nicht verallgemeinerbar, so daß die Nutzung von Transformationsprotokollen, die beispielsweise für die Sonnenblume ausgearbeitet worden sind, für andere Gattungen, wie Tagetes, nicht möglich ist.

Der vorliegenden Erfindung liegt daher die Aufgabe zugrunde, ein Verfahren zur Herstellung von transgenen, fertilen Pflanzen der Gattung Tagetes bereitzustellen.

Das erfindungsgemäße Verfahren eröffnet erstmals die Möglichkeit der Regeneration transformierter Pflanzenzellen der Gattung Tagetes zu fertilen, transgenen Pflanzen.

Das Verfahren für die Herstellung stabil transformierter Tagetespflanzen besteht aus folgenden Schritten: (a) der Anzucht der zu transformierenden Ausgangspflanze sowie Gewinnung des geeigneten Explantates; (b) dem Transfer von DNA-Sequenzen in Pflanzenzellen; (c) der Selektion transformierter Pflanzenzellen und (d) der Regeneration dieser transformierten Pflanzenzellen zu fertilen, transgenen Pflanzen, wobei für die Regeneration in der phase 1 ein Cytokinin bzw. Cytokininkonjugat und ein Auxin bzw. Auxinkonjugat und in der Phase 2 ein Auxin bzw. Auxinkonjugat und Gibberellinsäure GA3 eingesetzt werden.

Als Methoden zur Transformation sind sowohl der direkte als auch indirekte Gentransfer geeignet. Erfolgreich einsetzbar sind die Agrobakterien vermittelte Transformation unter Verwendung der verschiedensten Ausgangsexplantate, wie z.B. Kotyledonen, Blätter, Hypokotyle, Sprosse, Wurzeln, Kallus, reife und unreife Samen bzw. Blütengewebe. Dabei kann der Gentransfer sowohl durch alleinige Cokultivierung/Inkubation bzw. Benetzung mit dem Agrobakterienstamm als auch durch eine unterstützende Vakuuminfiltration der Explantate mit der entsprechenden Bakterienkultur erfolgen. Der Einsatz von Ammenkulturen als Helfer kann dabei von Vorteil sein. Jeder Agrobakterienstamm, der ein Ti oder Ri Plasmid mit den für den Transfer erforderlichen genetischen Informationen enthält, ist als Vektor für die Transformation geeignet. Geeignete Agrobakterienstämme sind EHA101[pEHA101], EHA105[pEHA105], LBA4404[pAL4404], C58C1[pMP90] und C58C1[pGV2260]. Auch virale Vektoren erscheinen für die Tagetestransformation geeignet. Weitere Methoden zur Übertragung von genetischem Material in Tagetes sind beispielsweise die Protoplastentransformation vermittelt durch Polyethylenglykol (PEG), die Elektroporation, die Sonikation oder Mikroinjektion, sowie die Transformation intakter Zellen oder Gewebe durch Mikro- oder Makroinjektion in Gewebe oder Embryonen, Gewebeelektroporation, Inkubation trockener Embryonen in DNA-haltiger Lösung, die Vakuuminfiltration von Samen, siehe auch Eds. Galun, E. and Breiman, A. In: Transgenic Plants, Imperial College Press, 1997. Der Einsatz der Partikelkanone für den Beschuß der verschiedensten Explantate, wie beispielsweise Blätter und Kallus, ist ebenfalls möglich.

Für die durch Agrobakterien vermittelte Transformation können alle Gentransfervektoren eingesetzt werden, die die für den Transfer der T-DNA notwendigen Bordersequenzen enthalten (linker und rechter Border, LB bzw. RB), ein oder mehrere selektierbare Marker- oder Reportergene unter Kontrolle geeigneter Promotoren sowie Terminatoren tragen und/oder weitere Nutz- oder Zielgene ebenfalls unter Kontrolle geeigneter transkriptioneller und translationeller regulatorischer Einheiten enthalten. Als selektierbare Marker- bzw. Reportergene sind sowohl die Gene bar/PAT, NPTII und DOG^{R}1 als auch beispielsweise die folgenden denkbar: das Chloramphenicolacetyltransferase (CAT) - Gen aus dem Transposon Tn9, das Octopinsynthase- und das Nopalinsynthasegen, beide aus der T-Region des Plasmides pTi, das Hygromycinphosphotransferasegen aus E.coli, das aroA Gen aus Salmonella typhimurium, welches die EPSP-Synthase codiert und dadurch Resistenz gegen Glyphosat vermittelt, das Glucuronidasegen aus E. coli, das Luciferasegene sowie das GFP-Gen in seinen bisher zur Verfügung stehenden Varianten, welches für ein selbstfluoreszierendes - ursprünglich ausschließlich grün fluoreszierendes - Protein codiert. Ferner können alle weiteren Gene die für Resistenz gegen Antimetabolite, Antibiotika oder Herbizide codieren als selektierbare Markergene verwendet werden, siehe auch Wilmink, A. and Dons, J.J.M. (Plant Mol. Biol. 11(1993), 165 - 185). Auch positive Selektionssysteme sind einsetzbar, wie beispielsweise der Erwerb der Fähigkeit einer Zuckerverwertung wie in WO 94/20627 beschrieben.

Vorzugsweise erfolgt der Transfer von DNA-Sequenzen in die zu transformierende Tagetespflanze bzw. deren Explantate durch Agrobacterium tumefaciens vermittelten Gentransfer.

Das erfindungsgemäße Regenerationsmedium enthält eine Saccharosekonzentration von 0,5 - 5 % Saccharose, vorzugsweise von 1 - 3 % Saccharose. Dabei kann Saccharose gegebenenfalls auch durch andere Zucker - u.a. Glucose oder Fructose - ersetzt werden.

Das erfindungsgemäße Regenerationsmedium enthält weiterhin Wachstumsregulatoren wie Auxine und/oder Auxinkonjugate, sowie Cytokinine und/oder Cytokininkonjugate. Als Auxine kommen sowohl natürliche als auch synthetische Auxine in Frage. Das natürliche Auxin ist Indolessigsäure (IAA), synthetische Auxine sind z.B. Indol-3-buttersäure (IBA), Naphth-1-ylessigsäure (NAA) und das Herbizid 2,4-Dichlorphenoxyessigsäure (2,4-D). Auch andere Herbizide mit Auxinwirkung sind als Wachstumsregulatoren denkbar. Auxinkonjugate sind Verbindungen des Auxins (IAA) mit Asparaginsäure, Glucose, Myoinositol und anderen Partnern. Auch bei den Cytokininen sind natürliche, wie z.B. Zeatin und synthetische Komponenten, wie 6-Benzylaminopurin (BAP) und 6-Furfurylaminopurin (Kinetin) gegebenenfalls einsetzbar. Zeatinribosid wird häufig als Cytokininkonjugat verwendet. Dabei können die Auxine und die Cytokinine jeweils als Einzelkomponenten als auch als Auxin- bzw. Cytokiningemische eingesetzt werden. Die Konzentration der einzelnen Wachstumsregulatoren liegt bei 0.05 - 10 mg/l, vorzugsweise bei 0.1 - 5 mg/l.

In einer besonderen Ausführungsform werden dem Kultivierungsmedium zunächst die Wachstumsregulatoren Benzylaminopurin, sowie Indolessigsäure für die Kultivierung der Explantate während und nach der Co-Kultivierung mit den Bakterien zugesetzt (Phase 1). Nach der Co-Kultivierung erfolgt der Transfer der Explantate auf das gleiche Basalmedium, das zusätzlich ein Antibiotikum, wie z.B. Carbenicillin, Timentin, Ticarcillin, Cefotaxime oder β-Bactyl zur Unterdrückung des Bakterienwachstums sowie das eigentliche selektive Agens zur Anreicherung des transgenen Zellmaterials enthält. Der Transfer auf frisches Medium derselben Zusammensetzung erfolgt jeweils nach 14 Tagen bis sich Sproßknospen und kleine Sprosse entwickelt haben (Dauer: ca. 4-6 Wochen). Anschließend wird für die weitere Sproßstreckung und Wurzelentwicklung ein Medium verwendet, das anstelle von Benzylaminopurin und Indolessigsäure die Wachstumsregulatoren Indol-3-buttersäure und die Gibberellinsäure GA₃ enthält (Phase 2).

Das erfindungsgemäße Regenerationsverfahren zur Herstellung von transgenen, fertilen Pflanzen der Gattung Tagetes ist u.a. anwendbar bei den Arten Tagetes (T.) patula, T. erecta, T. laxa, T. minuta, T. lucida, T. argentina cabrera, T. tenuifolia, T. lemmonii bzw. T. bipinata.

### Beispiel 1

Konstruktion des Binärplasmides pPTGI

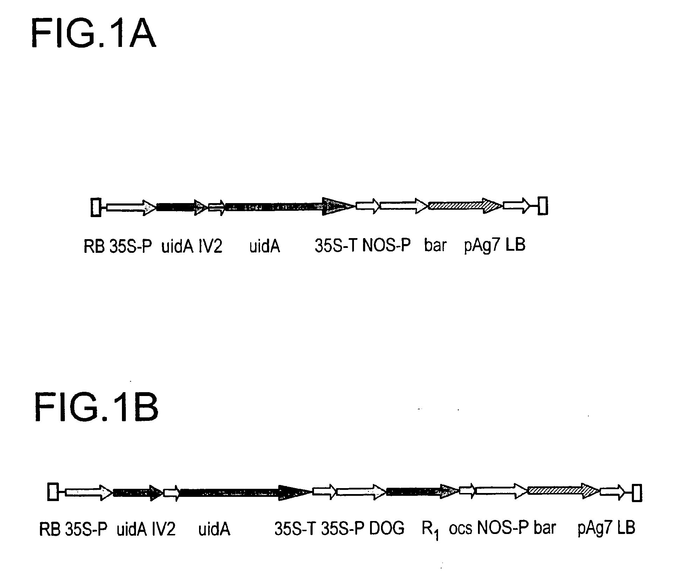

Für die Konstruktion des Binärplasmides pPTGI (Fig. 1A) wurde das Plasmid pGPTV-Bar (Becker, D. et al., Plant Mol. Biol. 20 (1992), 1195-1197) verwendet. Das promotorlose GUS-Gen dieses Plasmides wurde durch die Spaltung mit den Restriktionsenzymen EcoRI und SmaI entfernt. Die überstehenden Enden sind anschließend unter Verwendung von Klenow-Polymerase aufgefüllt und das Fragment mit einem ebenso behandelten PstI Fragment, welches das Glucuronidasegen mit einem Intron (GUS-IV2) enthält (Vancanneyt, Mol. Gen. Genet. 220 (1990), 245-250), ligiert worden.

Für die Konstruktion des Binärplasmides pPTGIDOG wurde das DOG^{R}1 Gens mit seinem angrenzenden 35S Promotor- und ocs-Terminatorbereich durch PCR amplifiziert. Dazu diente das pBinAR-DOG^{R}1 Konstrukt, das von Sonnewald und Ebneth, EP0807836 beschrieben worden ist, als Mustersequenz. Für die Amplifikation sind die beiden Primer 35SXbaI und OcsXbaI eingesetzt worden. 35SXbaI ist zu den Nukleotiden 1 bis 24 des 35S Promotors des Plasmides pBinAR (Höfgen und Willmitzer (Plant Science) 66 (1990), 221-230) homolog und enthält zusätzlich eine XbaI Erkennungsregion am 5' Ende (5'-ATTCTAGACATGGAGTCAAAGATTCAAATAGA-3'). OcsXbaI ist den letzten 24 Nukleotiden der ocs Terminatorregion des Plasmides pBinAR homolog und enthält einen zusätzlichen XbaI Restriktionsort (5'-ATTCTAGAGGACAATCAGTAAATTGAACGGAG-3'). Das amplifizierte Fragment wurde mit Klenow-Polymerase von seinen überhängenden Einzelstrangenden befreit, mit HindIII Linkern ligiert und in das Plasmid pBS+ (Stratagene, Californien, USA) cloniert. Nach Sequenzanalyse ist das komplette Fragment, das den 35S Promotor, das DOG^{R}1 Gen und die ocs-Terminatorregion enthielt, als HindIII Fragment in den HindIII geschnittenen Vektor pPTGI kloniert worden. Fig. 1 zeigt die Genkarten der T-DNAs der beiden Binärplasmide pPTGI (5007 bp) (Fig. 1A) und pPTGI-DOG^{R}1 (6209 bp) (Fig. 1B), die für die Transformationen von Tagetes verwendet wurden. Die Abkürzungen haben folgende Bedeutung:
- 35S-P:: CaMV 35S Promotor
- 35S-T:: CaMV 35S Terminator
- NOS-P:: Nopalinsynthasepromotor
- uidA:: β-Glucuronidasegen
- bar:: Phosphinothricinacetyltransferasegen
- LB:: Left border
- RB:: Right border
- IV2:: 2. Intron des Gens ST-LS1
- pAg7:: Gen 7-Poly(A)signal
- DOG^{R}1:: 2-DOG-phosphat-Phosphatase Gen aus Saccharomyces cerevisiae S288c
- ocs:: Terminator der Octopinsynthase

### Beispiel 2

Ermittlung der Toxizität von Phosphinothricin (PPT)

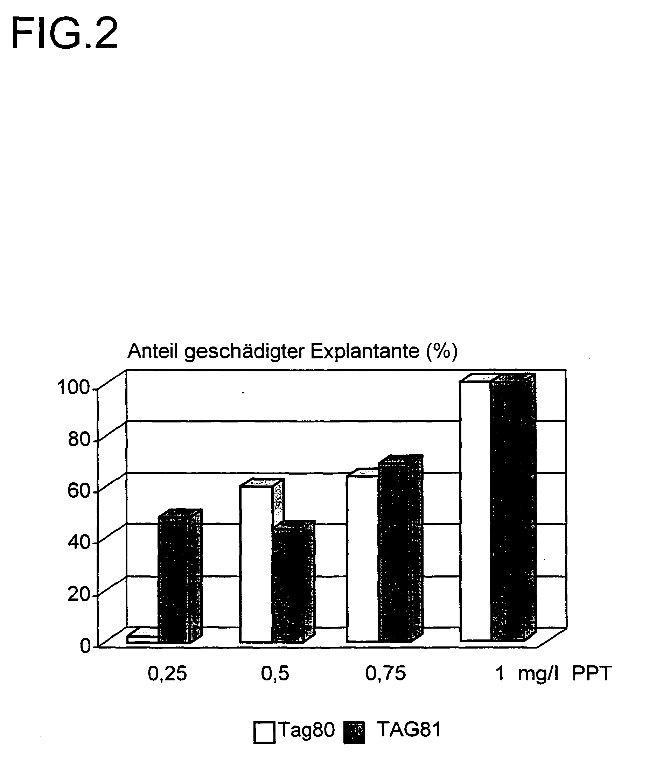

Blätter von in vitro etablierten Tagetes patula Pflanzen der Genotypen TAG 80 und TAG 81 (Genbank, IPK Gatersleben) wurden quer zur Mittelrippe in 10 bis 60 mm² große Scheiben geschnitten, auf MS-Medium mit 2 % Saccharose, 3 mg/l Benzylaminopurin (BAP) und 1 mg/l Indolessigsäure (IAA) und unterschiedlichen Konzentrationen an PPT gelegt. Die Inkubation fand in einem 16/8 h Licht/Dunkel Rhythmus bei ca. 50 µE/m²s und 21 bis 24°C für 2 Wochen statt. Anschließend wurde der Anteil geschädigter Explantate an der Gesamtzahl getesteter Explantate erfaßt (Fig. 2). Konzentrationen ab 1 mg/l PPT führten bei den beiden getesteten Tagetes patula Genotypen zum kompletten Absterben aller Explantate, deshalb wurde diese Konzentration für die Selektion nach der Cokultivierung verwendet.

### Beispiel 3

### Tagetes erecta Transformation

In vitro Sämlingsexplantate von Tagetes erecta TAG 76 (Genbank, IPK, Gatersleben) wurden als primäres Targetmaterial genutzt. Zur Oberflächensterilisation wurde das Saatgut für 5 Minuten in 70%igem Ethanol inkubiert und anschließend gründlich mit sterilem destillierten Wasser gewaschen. Danach wurden die Samen mit Filterpapier abgetrocknet, auf festes MS - Medium (Murashige and Skoog (1962) Physiol. Plant. 15, 473-497) gelegt und für 3 bis 12 Wochen im 16/8 h Licht/Dunkel Rhythmus bei ca. 50 µE/m²s und bei 21 bis 24°C inkubiert. Alle Blätter (außer Kotyledonen), die sich während dieser Zeit gebildet hatten, wurden geerntet und quer zur Mittelrippe geschnitten. Die dadurch entstandenen Blattexplantate mit einer Größe von 10 bis 60 mm² wurden im Verlaufe der Präparation in flüssigem MS-Medium bei Raumtemperatur für maximal 2 h aufbewahrt.

Die Agrobacterium tumefaciens Kultur EHA105[pPTGI] wurde über Nacht in YEB (0,1 % Hefeextrakt, 0,5 % Rindfleischextrakt, 0,5 % Pepton, 0,5 % Saccharose, 0,5 % Magnesiumsulfat x 7 H₂O) mit 25 mg/l Kanamycin durch Animpfen einer Einzelkolonie bei 28°C für 16 bis 20 h angezogen. Anschließend wurde die Bakteriensuspension durch Zentrifugation bei 6000 g für 10 min geerntet und derart in flüssigem MS Medium resuspendiert, daß eine OD von ca. 0,3 bis 0,8 entstand.

Unmittelbar vor der Co-Kultivierung ist das MS-Medium, in dem die Blätter aufbewahrt worden sind, durch die Bakteriensuspension ersetzt worden. Die Inkubation der Blättchen in der Agrobakteriensuspension erfolgte für 30 min unter leichtem Schütteln bei Raumtemperatur. Anschließend wurden die infizierten Explantate auf ein mit 0,8 % Plant Agar (Duchefa, NL) verfestigtes MS-Medium mit 2 % Saccharose und mit 3 mg/l Benzylaminopurin (BAP) sowie 1 mg/l Indolylessigsäure (IAA) aufgelegt. Die Applikation der Wachstumsregulatoren entspricht einer von Kothari und Chandra (1984, HortScience 19, 703-705) entwickelten Kombination. Die Orientierung der Blätter auf dem Medium ist bedeutungslos. Die Kultivierung der Explantate fand für 6 d unter denselben Bedingungen statt wie für die Keimung der Tagetessamen beschrieben wurde. Anschließend wurden die co-kultivierten Explantate auf frisches MS-Medium mit den gleichen Wachstumsregulatoren übertragen, wobei dieses zweite Medium zusätzlich 250 mg/l β-Bactyl und 1 mg/l PPT enthielt.

In Abständen von 14 Tagen erfolgte der Transfer der Explantate auf frisches Medium bis sich Sproßknospen und kleine Sprosse entwickelt hatten, die dann auf das gleiche Basalmedium einschließlich β-Bactyl und PPT aber mit einer veränderten Kombination an Wachstumsregulatoren, nämlich 0,5 mg/l IBA und 0,5 mg/l GA₃, zur Bewurzelung übertragen wurden. Bewurzelte Sprosse konnten ins Gewächshaus überführt werden.

### Beispiel 4

### Tagetes patula Transformation

In vitro etablierte Tagetes patula TAG80 Pflanzen (Genbank, IPK, Gatersleben) dienten als Targetmaterial für die Transformation. Sie wurden routinemäßig durch Sproßspitzenvermehrung unter Verwendung von festem MS - Medium, dem die Wachstumsregulatoren IBA (0,5 mg/l) und GA₃ (0,5 mg/l) zugesetzt worden waren, erhalten bzw. vermehrt. Blätter dieser Pflanzen wurden mit dem Agrobacterium tumefaciens Stamm EHA105[pPTGI-DOG^{R}1] für 4, 6 und 8 Tage co-kultiviert. Die Versuchsdurchführung sowie die verwendeten Regenerationsbedingungen entsprachen denen unter Beispiel 3 beschriebenen. Potentielle transgene Sprossregenerate wurden in Gegenwart von 1 mg/l PPT bewurzelt.

### Beispiel 5

### Test auf Transgenizität

### A. Glucuronidasenachweis

Blätter und Wurzelsegmente der regenerierten in vitro Pflanzen wurden einem qualitativen Glucuronidase (GUS)-Enzym-Nachweis unterzogen, in dem sie in einem 100 mM Natriumphosphatpuffer, pH 7,0, der 10 mM EDTA, 0,1 % Triton X100, 10 mM DTT enthielt, mit dem GUS Substrat 5-Bromo-4-chloro-3-indolyl-β-D-glucuronic acid (X-GlcA) für 1 min unter Vakuum infiltriert und anschließend für ca. 15 h bei 37°C inkubiert wurden. Anschließend erfolgte die Entfärbung der Explantate mit 70%igem Ethanol bei Raumtemperatur. Blätter und Wurzeln zeigten eine intensive Blaufärbung, was die Expression des Reportergens in Tagetes beweist.

### B. Genomische PCR Analyse

Von den zu testenden potentiellen transgenen Pflanzen sowie von 3 nichttransformierten Wildtyppflanzen wurde genomische DNA wie folgt isoliert: Ca. 100 mg Blattmaterial wurde in einem Reaktionsgefäß in flüssigem Stickstoff eingefroren und anschließend mit einem Homogenisator aufgeschlossen. Die Extraktion erfolgte in 900 µl Extraktionspuffer (100 mM Tris-HCl, pH 8,0, 500 mM NaCl, 50 mM Na₂EDTA, 10 mM Mercaptoethanol). Nach Zugabe von 100 µl 20%iger SDS-Lösung wurde gemischt und für 10 min bei 65°C inkubiert. Nach Zugabe von 200 µl 5 M Kaliumacetatlösung schloß sich eine 20 minütige Inkubation bei 4°C in einem Eisbad an. Das in dieser Zeit entstandene Präzipitat wurde durch Zentrifugation bei 12000 g für 15 min entfernt und die im Überstand enthaltene DNA wurde durch Zugabe von 800 µl eiskaltem Isopropanol bei -20°C innerhalb von 30 min ausgefällt. Die Proben wurden zentrifugiert, um die DNA zu sammeln, die anschließend mit 70%igem, eiskaltem Ethanol gewaschen, luftgetrocknet und in 25 µl 10 mM Tris-HCl, pH 8,0, 1 mM EDTA (TE) mit 0,2 mg/ml RNase A resuspendiert wurde. 3 µl der so isolierten DNA wurden für die PCR Reaktion eingesetzt.

Das DOG^{R}1 Gen umfaßt 741 bp und wurde durch die Verwendung der beiden Primer DOG^{R}1-1 und DOG^{R}1-2 amplifiziert. DOG^{R}1-1 ist zu den Nukleotiden 1 bis 26 des DOG^{R}1 Gens homolog und enthält 10 zusätzliche Nukleotide, die die Restriktionserkennungsorte BamHI und NcoI umfassen (5'-ATGGATCCCCATGGCAGAATTTTCAGCTGATCTATG-3'). DOG^{R}1-2 ist zu den Nukleotiden 720 bis 741 des DOG^{R}1 Gens homolog und enthält 11 zusätzliche Nukleotide, die den Restriktionserkennungsort SalI beinhalten (5'-ATGTCGACTACTCAGGCCCTTGTCAAAGGGTTG-3'). Der PCR-Ansatz erfolgte nach den Angaben des Herstellers Takara (Japan). Folgende Inkubationsschritte liefen ab:
1. 94°C - 4 min
2. 94°C - 15 sec
3. 58°C - 30 sec
4. 72°C - 1 min
4 malige Wiederholung der Schritte 2 - 4
5. 94°C - 15 sec
6. 56°C - 30 sec
7. 72°C - 1 min
4 malige Wiederholung der Schritte 5 - 7
8. 94°C - 15 sec
9. 52°C - 30 sec
10. 72°C - 1 min
24 malige Wiederholung der Schritte 8 - 10
11. 72°C - 10 min
12. 4°C - Ende der Reaktion

Im Anschluß an die PCR Reaktion sind 5 µl der einzelnen Proben mit 5 µl Stopperlösung (10 mM Tris HCl, pH 8,0, 50 % Glycerin, 0,05 % SDS, 0,2 % Xylencyanol) versetzt und in einem 1%igen Agarosegel aufgetrennt worden. Nach Ablauf der elektrophoretischen Trennung bei 100 V für 30 min wurde das Gel unter UV-Licht ausgewertet. Tabelle 1 enthält die Ergebnisse der Analysen zur Transgenizität potentieller Tagetes patula Transformanten. All diese Pflanzen wurden in Gegenwart von 1 mg/l PPT regeneriert. Es wurden nur solche Pflanzen für die PCR-Analyse verwendet, die in Gegenwart des Herbizids wiederholt Wurzeln bilden konnten. In all diesen Pflanzen konnte das DOG^{R}1 Gen nachgewiesen werden. Erstaunlicherweise war nur bei wenigen Pflanzen GUS Aktivität in den Blättern nachweisbar.

**Tabelle 1: Untersuchung potentieller transgener Tagetes patula Pflanzen, die mit dem Binärplasmid pPTGI-DOG^{R}1 transformiert worden sind (siehe Beispiel 4).**

| Pflanzen- Nr. | Dauer der Co-Kultur (d) | Resistenz/ Wiederbewurzelung^{a)} | GUS-Aktivität^{b)} | genomische PCR (DOG-^{R}1-Primer)^{c)} |
|---|---|---|---|---|
| 1 | 6 | - | - | n.b. |
| 2 | 6 | - | - | n.b. |
| 3 | 6 | - | - | n.b. |
| 4 | 6 | + | - | + |
| 5 | 6 | (+) | - | + |
| 6 | 6 | (+) | - | + |
| 7 | 6 | (+) | - | + |
| 8 | 6 | - | - | n.b. |
| 9 | 6 | ++ | - | + |
| 10 | 6 | ++ | - | + |
| 11 | 6 | ++ | - | + |
| 12 | 6 | - | - | n.b. |
| 13 | 6 | - | - | n.b. |
| 14 | 6 | - | - | n.b. |
| 15 | 6 | + | - | + |
| 16 | 6 | + | - | + |
| 17 | 6 | ++ | (+) | + |
| 18 | 6 | - | - | n.b. |
| 19 | 6 | (+) | - | - |
| 20 | 4 | - | - | n.b. |
| 21 | 4 | - | - | n.b. |
| 22 | 4 | ++ | (+) | + |
| 23 | 4 | + | - | + |
| 24 | 8 | - | - | n.b. |
| 25 | 8 | - | - | n.b. |
| 26 | 8 | (+) | - | + |
| 27 | 8 | ++ | (+) | + |
| 28 | 8 | - | - | n.b. |
| 29 | 8 | (+) | (+) | + |
| 30 | 8 | ++ | (+) | + |

| | | | | |
|---|---|---|---|---|
| Zeichenerklärung: a) - Der Sproß dieser Pflanze kann in Gegenwart von 1 mg/l PPT keine Wurzeln ausbilden, zeigt nur kümmerliches Wachstum und stirbt ab. (+) Der Sproß zeigt nur ein geringes Wurzelbildungsvermögen. Die Pflanze wächst nur wenig. + Der Sproß zeigt eine deutliche Wurzelbildung. Die Pflanze wächst normal. ++ Der Sproß bildet in kurzer Zeit ein kräftiges Wurzelsystem. Die Pflanze wächst ohne Beeinträchtigung in Gegenwart von 1 mg/l PPT. b) - Es ist keine GUS Aktivität nachweisbar. (+) Es zeigt sich eine schwache GUS Aktivität in Form von kleinen, blau gefärbten Punkten auf der Blattoberfläche. c) n.b. nicht bestimmt + Nach PCR Reaktion und anschließender gelelektrophoretischer Auftrennung läßt sich eine deutliche Bande von ca. 750 bp nachweisen. In Kontrollansätzen, für die die genomische DNA von Tagetes Wildtyppflanzen eingesetzt worden ist, war diese Bande nicht nachzuweisen. | | | | |

### Beispiel 6

### Nachkommenschaftsanalyse

Für die Nachkommenschaftsanalyse wurde die transgene Tagetes erecta TAG 76 Pflanze Nr. 2 eingesetzt. Die Herstellung dieser Pflanze wurde unter Beispiel 3 beschrieben.

Saatgut dieser Pflanze sowie von einer unbehandelten Wildtyppflanze wurde in Pflanzschalen (ca. 30 x 60 cm) ausgesät und unter Gewächshausbedingungen bei ca. 25°C kultiviert. Nach 12 Tagen hatten die Keimlinge ca. 3 bis 4 Blätter gebildet und eine Höhe von ca. 5 bis 10 cm erreicht. Sie wurden dann dreimal in Abständen von je einem Tag mit einer 1:1000 verdünnten Lösung des Herbizids Basta besprüht. Vier Tage nach der letzten Sprühapplikation des Herbizids erfolgte die Auswertung des Versuches. Alle 83 besprühten Wildtypkeimlingen zeigten massive Schäden oder waren bereits abgestorben. Bei der Keimlingsnachkommenschaft der transgenen Pflanze Nr. 2 zeigten von 190 besprühten Pflanzen 134 keinerlei Schäden und 56 waren geschädigt bzw. starben ab. Bei diesem Spaltungsverhältnis von ca. 2,5 resistenten : 1 sensitiven Pflanze liegt eine mendelnde Vererbung, die im Idealfall 3 : 1 ergeben müßte, nahe.

## Patentansprüche

1. Verfahren für die Herstellung stabil transformierter, fertiler Pflanzen der Gattung Tagetes, **dadurch gekennzeichnet, daß** folgende Schritte enthalten sind;
(a) Anzucht der zu transformierenden Ausgangspflanze sowie Gewinnung des geeigneten Explantates
(b) Transfer von DNA-Sequenzen in Pflanzenzellen
(c) Selektion transformierter Pflanzenzellen
(d) Regeneration fertiler, transgener Pflanzen, wobei für die Regeneration in der Phase 1 ein Cytokinin bzw. Cytokininkonjugat und ein Auxin bzw. Auxinkonjugat und in der Phase 2 ein Auxin bzw. Auxinkonjugat und Gibberellinsäure GA3 eingesetzt werden.

2. Verfahren gemäß Anspruch 1, **dadurch gekennzeichnet dass** für die Regeneration in der Phase 1 Benzylaminopurin und Indolessigsäure und in der Phase 2 Indolbuttersäure und Gibberellinsäure GA3 eingesetzt werden.

3. Verfahren gemäß Anspruch 1 oder 2, **dadurch gekennzeichnet, daß** der Transfer von DNA-Sequenzen in die zu transformierende Pflanze bzw. deren Explantate durch Agrobakterien erfolgt.

4. Verfahren gemäß Anspruch 3, **dadurch gekennzeichnet, daß** Blätter als Explantate verwendet werden.

5. Verfahren gemäß Anspruch 3 oder 4, **dadurch gekennzeichnet, daß** eine 2 bis 8 tägige Co-Kultivierung mit Agrobakterien erfolgt.

6. Verfahren gemäß den Ansprüchen 3 bis 5, **dadurch gekennzeichnet, daß** es sich bei der verwendeten Agrobakterienart um Agrobacterium tumefaciens handelt.

7. Verfahren gemäß den Ansprüchen 3 bis 6, **dadurch gekennzeichnet, daß** während der Co-Kultivierung ein Medium verwendet wird, das Wachstumsregulatoren enthält.

8. Verfahren gemäß den Ansprüchen 1 bis 7, **dadurch gekennzeichnet, daß** für die Selektion der transformierten Zellen Herbizid-, Antibiotika- oder Antimetabolitresistenzen eingesetzt oder positive Selektionsverfahren genutzt werden.

9. Verfahren gemäß Anspruch 8, **dadurch gekennzeichnet, daß** für die Selektion Phosphinothricin (PPT) eingesetzt wird.

10. Verfahren gemäß den Ansprüchen 1 bis 9, **dadurch gekennzeichnet, daß** die Art Tagetes erecta bzw. Tagetes patula transformiert wird.

11. Transgene, fertile Tagetespflanze.

12. Transgene, fertile Tagetespflanze hergestellt durch ein Verfahren nach den Ansprüchen 1 bis 10.

13. Transgene Blütenblätter einer Tagetespflanze gemäß Anspruch 11 oder 12.

14. Transgene Samen einer Tagetespflanze gemäß Anspruch 11 oder 12.

## Claims

1. A method for the generation of stably transformed fertile plants of the genus Tagetes, which comprises the following steps:
(a) growing the starting plant to be transformed and obtaining the suitable explant,
(b) transferring DNA sequences into plant cells,
(c) selecting transformed plant cells,
(d) regenerating fertile transgenic plants, the regeneration employing a cytokinin or cytokinin conjugate and an auxin or auxin conjugate in phase 1 and an auxin or auxin conjugate and gibberellic acid GA3 in phase 2.

2. The method according to claim 1 wherein the regeneration employs benzylaminopurine and indole acetic acid in phase 1 and indole butyric acid and gibberellic acid GA3 in phase 2.

3. The method according to claim 1 or 2 wherein DNA sequences are transferred into the plant to be transformed or explants thereof by means of agrobacteria.

4. The method according to claim 3 wherein leaves are used as explants.

5. The method according to claim 3 or 4 wherein cocultivation with agrobacteria takes place for 2-8 days.

6. The method according to claims 3 to 5 wherein the Agrobacterium species used is Agrobacterium tumefaciens.

7. The method according to claims 3 to 6 wherein a medium is used during the cocultivation which comprises growth regulators.

8. The method according to claims 1 to 7 wherein herbicide, antibiotic or antimetabolite resistances are employed for selecting the transformed cells or positive selection methods are used.

9. The method according to claim 8 wherein phosphinothricin (PPT) is employed for the selection.

10. The method according to claims 1 to 9 wherein the species Tagetes erecta or Tagetes patula is transformed.

11. A transgenic fertile Tagetes plant.

12. A transgenic fertile Tagetes plant generated by a method according to claims 1 to 10.

13. Transgenic petals of a Tagetes plant according to claim 11 or 12.

14. Transgenic seed of a Tagetes plant according to claim 11 or 12.

## Revendications

1. Procédé de production de plantes fertiles, transformées de manière stable, de l'espèce des tagètes, **caractérisé en ce qu'**il inclut les étapes suivantes :
(a) culture des plantes de départ devant être transformées, et production de l'explantat approprié
(b) transfert de séquences d'ADN dans des cellules végétales
(c) sélection de cellules végétales transformées
(d) régénération de plantes transgéniques fertiles, au cours de laquelle sont utilisés respectivement, pour la régénération dans la phase 1, de la cytokinine ou une liaison conjuguée de cytokinine, et de l'auxine ou une liaison conjuguée d'auxine et dans la phase 2, de l'auxine ou une liaison conjuguée d'auxine, et de l'acide gibbérellique GA3.

2. Procédé selon la revendication 1, **caractérisé en ce que** sont utilisés respectivement, pour la régénération dans la phase 1, de la benzylaminopurine et de l'acide indol-acétique, et pour la phase 2, de l'acide indolylbutyrique et de l'acide gibbérellique GA3.

3. Procédé selon la revendication 1 ou 2, **caractérisé en ce que** le transfert de séquences d'ADN dans les plantes devant être transformées ou leurs explants est réalisé par l'intermédiaire d'agrobactéries.

4. Procédé selon la revendication 3, **caractérisé en ce que** des feuilles sont utilisées en tant qu'explants.

5. Procédé selon la revendication 3 ou 4, **caractérisé en ce qu'**une co-culture d'une durée de 2 à 8 jours avec des agrobactéries est réalisée.

6. Procédé selon l'une quelconque des revendications 3 à 5, **caractérisé en ce que** le type d'agrobactérie utilisé, et dont il s'agit, est agrobacterium tumefaciens.

7. Procédé selon l'une quelconque des revendications 3 à 6, **caractérisé en ce que**, pendant la co-culture, un milieu contenant des régulateurs de croissance est utilisé.

8. Procédé selon l'une quelconque des revendications 1 à 7, **caractérisé en ce que** pour la sélection des cellules transformées, des résistances aux herbicides, aux antibiotiques ou aux antimétabolites sont mises en oeuvre, ou bien des procédés de sélection positifs sont utilisés.

9. Procédé selon la revendication 8, **caractérisé en ce que**, pour la sélection, de la phosphinothricine (PPT) est utilisée.

10. Procédé selon l'une quelconque des revendications 1 à 9, **caractérisé en ce que** l'espèce tagetes erecta ou tagetes patula est transformée.

11. Plante fertile transgénique tagète.

12. Plante fertile transgénique tagète produite par un procédé selon l'une quelconque des revendications 1 à 10.

13. Pétales transgéniques d'une plante tagète selon la revendication 11 ou 12.

14. Semences transgéniques d'une plante tagète selon la revendication 11 ou 12.
